# EUROPÄISCHE PATENTSCHRIFT

(11) **EP 1 446 061 B1**
(45) Veröffentlichungstag und Bekanntmachung des Hinweises auf die Patenterteilung: **01.03.2006**
(21) Anmeldenummer: 01982040.6
(22) Anmeldetag: 22.11.2001
(51) Int. Cl.: A61B 17/70

(54) **VORRICHTUNG ZUR VERBINDUNG EINES LÄNGSTRÄGERS MIT EINEM KNOCHENFIXATIONSMITTEL**
DEVICE FOR JOINING A LONGITUDINAL SUPPORT WITH A BONE FIXATION MEANS
DISPOSITIF PERMETTANT D'ASSEMBLER UN SUPPORT LONGITUDINAL ET UN MOYEN DE FIXATION OSSEUSE

(43) Veröffentlichungstag der Anmeldung: 18.08.2004
(73) Patentinhaber: Synthes AG Chur, 7002 Chur (CH)
(72) Erfinder: DONATH, Raoul, 79639 Grenzach-Wyhlen (DE)
(74) Vertreter: Lusuardi, Werther
(86) Internationale Anmeldenummer: PCT/CH2001/000682
(87) Internationale Veröffentlichungsnummer: WO 2003/043511

(56) Entgegenhaltungen:
- EP-A- 1 064 885
- FR-A- 2 729 291
- US-A- 5 429 639
- US-A- 6 063 089
- US-A- 6 077 262

## Beschreibung

Die Erfindung bezieht sich auf eine Vorrichtung zur Verbindung eines Längsträgers mit einem Knochenfixationsmittel, insbesondere einer Pedikelschraube gemäss dem Oberbegriff des Patentanspruchs 1. Eine solche Vorrichtung ist z.B. aus der EP-A-1064885 bekannt.

Aus dem Stand der Technik sind bereits einige Vorrichtungen zur Verbindung von Pedikelschrauben mit Längsträgern für die Wirbelsäulenfixation bekannt.

Eine solche Verbindungsvorrichtung ist in der US 5,584,834 ERRICO offenbart. Diese bekannte Erfindung beinhaltet eine Vorrichtung, die es auf einfache Weise gestattet, eine Pedikelschraube oder allgemeiner ein Knochenverankerungselement mit einem Längsträger zu verbinden. Die Verbindungsvorrichtung besteht im wesentlichen aus einem zylinderförmigen Zentralkörper, welcher an seinem unteren Ende mit einer geschlitzten, aussen konischen Spannzange mit einer hohlkugelförmigen Kavität zur Aufnahme eines kugelförmigen Kopfes eines Knochenverankerungselementes versehen ist und an seinem oberen Ende ein Aussengewinde aufweist. In seinem Mittelteil ist dieser Zentralkörper mit einer quer zu seiner Zentralachse verlaufenden seitlich offenen Bohrung zur Aufnahme eines Längsträgers versehen. Über diesen Zentralkörper werden eine untere Hülse mit einem zum Konus des Zentralkörpers korrespondierenden Innenkonus und eine obere Hülse mit einer gegen die untere Hülse hin offenen Durchgangsöffnung geschoben. Beide Hülsen sind im montierten Zustand mittels einer Mutter, welche über das Aussengewinde am Zentralkörper schraubbar ist, gegen unten pressbar. Zwischen unterer und oberer Hülse wird der Längsträger eingeführt, welcher durch die Durchgangsöffnung in der oberen Hülse seitlich geführt wird. Beim Blockieren der Verbindungsvorrichtung wird beim Anziehen dieser Mutter die obere Hülse gegen unten gepresst, wodurch der in der Durchgangsöffnung eingeführte Längsträger auf die untere Hülse drückt, deren Innenkonus in der Folge über den Aussenkonus der Spannzange geschoben wird. Das übereinanderschieben der Konusse bewirkt ein Komprimieren der Spannzange und somit eine Blockierung des darin eingespannten Kugelkopfes. Nachteilig an dieser bekannten Vorrichtung ist einerseits die wegen der kugelgelenkartigen Verbindung zwischen dem sphärischen Kopf des Knochenverankerungselementes und der Spannzange entstehende grosse Bauhöhe des Verbindungsteiles sowie andererseits der zum Anziehen der über das Aussengewinde am Zentralkörper schraubbaren Mutter benötigte Platzbedarf für das chirurgische Instrument.

Hier will die Erfindung Abhilfe schaffen. Der Erfindung liegt die Aufgabe zugrunde, eine Vorrichtung zu schaffen, welche einen möglichst geringen Platzbedarf erfordert und zudem mit einem minimalen Bedarf an chirurgischen Instrumenten und Arbeitsvorgängen implantierbar ist. Insbesondere soll ein chirurgisches Instrument einsetzbar sein, dessen Durchmesser nicht grösser als der Durchmesser des Implantates ist, um somit eine möglichst geringe Schädigung der umliegenden Weichteile zu gewährleisten.

Die Erfindung löst die gestellte Aufgabe mit einer Vorrichtung zur Verbindung eines Längsträgers mit einem Knochenfixationsmittel, insbesondere einer Pedikelschraube, welche die Merkmale des Anspruchs 1 aufweist.

Die erfindungsgemässe Vorrichtung umfasst im wesentlichen ein Verbindungsteil mit einer Verschlusskappe und ein Spannmittel. Das Verbindungsteil ist mit einem zur Zentralachse koaxialen Hohlraum versehen, welcher am oberen und am unteren Ende des Verbindungsteiles offen ist. Der Hohlraum ist gegen das untere Ende des Verbindungsteiles mittels eines Absatzes verjüngt. Ein am oberen Ende des Verbindungsteiles offener, quer zur Zentralachse des Verbindungsteiles verlaufender Kanal durchdringt das Verbindungsteil diametral, so dass beispielsweise ein zu einem Wirbelsäulenfxationssystem zählender Längsträger im Kanal aufnehmbar ist und orthogonal zur Zentralachse verläuft, während ein Knochenfixationsmittel, beispielsweise eine Pedikelschraube parallel zur Zentralachse durch den Hohlraum durchführbar ist, bis der Schraubenkopf der Pedikelschraube durch den Absatz im Hohlraum axial arretiert wird. Die Verschlusskappe weist einen zweiten, ebenfalls quer zur Zentralachse angeordneten und gegen das vordere Ende der Verschlusskappe offenen Kanal auf, worin bei auf dem Verbindungsteil montierter Verschlusskappe der Längsträger aufgenommen wird. Durch diesen zweiten Kanal wird die Verschlusskappe von ihrem vorderen Ende bis zum Boden des zweiten Kanals in zwei quer zur Zentralachse elastisch deformierbare Segmente aufgeteilt. Die Spannmittel sind am hinteren Ende der Verschlusskappe mit dieser verbindbar und dienen zum Blockieren eines Längsträgers und eines Knochenfixationsmittels im Verbindungsteil. Zur Befestigung der Verschlusskappe am Verbindungsteil sind aussen am Verbindungsteil und im Hohlraum der Verschlusskappe zueinander komplementäre, ineinander einschnappbare Arretiermittel angebracht.

In der bevorzugten Ausführungsform der erfindungsgemässen Vorrichtung sind die Arretiermittel auf der Peripherie des Verbindungsteiles und ebenfalls peripher im Hohlraum in der Verschlusskappe angeordnet. Vorzugsweise bestehen die Arretiermittel aus Erhebungen am Verbindungsteil und dazu komplementären Vertiefungen im Hohlraum der Verschlusskappe.

In einer anderen Ausführungsform der erfindungsgemässen Vorrichtung umfasst der Absatz, welcher den Hohlraum am vorderen Ende der Verschlusskappe verjüngt, eine planare Auflagefläche. Diese Auflagefläche dient zur Auflage beispielsweise des Schraubenkopfes einer Pedikelschraube. Anstelle der planaren Ausgestaltung kann diese Auflagefläche auch sphärisch oder mehrere konzentrische Stufen aufweisend ausgestaltet sein.

Weitere vorteilhafte Ausgestaltungen der Erfindung sind in den abhängigen Ansprüchen gekennzeichnet.

Die durch die Erfindung erreichten Vorteile sind im wesentlichen darin zu sehen, dass dank der erfindungsgemässen Vorrichtung:
- eine sehr niedrige Bauhöhe des Verbindungsteiles mit Verschlusskappe erreichbar ist;
- die Vorrichtung während der Implantation mittels eines einzigen Verschlussmechanismus blockierbar ist; und
- Instrumente verwendbar sind, deren Durchmesser kleiner als sind als die Implantate, so dass auf grosse, traumatisierende Zugänge beim Patienten verzichtet werden kann und die Vorrichtung für die minimalinvasive oder navigierte Chirurgie prädestiniert ist. Auch bieten diese Implantate die Möglichkeit zur Versorgung der Patienten bei Anwendungen von thoraskopischen Zugängen.

In einer weiteren Ausführungsform der erfindungsgemässen Vorrichtung umfasst diese Sicherungsmittel, mittels welcher der Hohlraum im Verbindungsteil zwischen dem hinteren Ende des Schraubenkopfes und dem oberen Ende des Verbindungsteiles verengt wird. Dadurch wird das Knochenfixationsmittel im Verbindungsteil gegen Herausfallen am oberen Ende des Verbindungsteiles gesichert.

Durch diese Ausgestaltung mit Sicherungsmitteln ist der Vorteil erreichbar, dass vormontierte Implantate verwendbar sind, so dass ein Zeitverlust im Operationsraum vermeidbar ist und das Gefahrenpotential z.B. einer Verwechslung oder Falschmontage erheblich verringert wird.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 einen Längsschnitt durch die bevorzugte Ausführungsform der erfindungsgemässen Vorrichtung;
Fig. 2a eine Aufsicht auf das Verbindungsteil der in Fig. 1 dargestellten Ausführungsform der erfindungsgemässen Vorrichtung;
Fig. 2b eine Aufsicht auf die in Fig. 1 dargestellte Ausführungsform der erfindungsgemässen Vorrichtung;
Fig. 3 einen Schnitt durch eine andere Ausführungsform der erfindungsgemässen Vorrichtung;
Fig. 4 eine Aufsicht auf die in Fig. 3 dargestellte Ausführungsform der erfindungsgemässen Vorrichtung;
Fig. 5 einen Schnitt durch eine weitere Ausführungsform der erfindungsgemässen Vorrichtung; und
Fig. 6 einen Schnitt durch wiederum eine weitere Ausführungsform der erfindungsgemässen Vorrichtung.

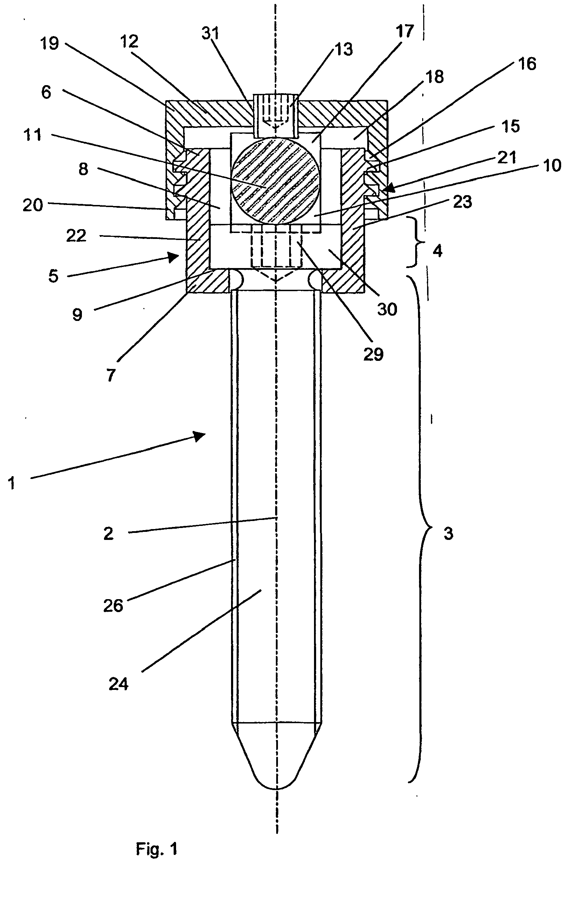

In Fig. 1 ist ein als Pedikelschraube ausgeführtes Knochenfixationsmittel 1 zusammen mit einem Verbindungsteil 5, einem Längsträger 11, einer vom oberen Ende 6 des Verbindungsteiles 5 koaxial über dieses geschobenen Verschlusskappe 12 und einem mit der Verschlusskappe 12 verbundenen Spannmittel 13 dargestellt. Das vordere Segment 3 des Knochenfixationsmittels 1 ist als Schraubenschaft 24 mit Aussengewinde 26 ausgebildet, während das hintere Segment 4 als kreiszylindrischer Schraubenkopf 30 ausgebildet ist. Das als Pedikelschraube ausgebildete Knochenfixationsmittel 1 ist mittels des Aussengewindes 26 am Schraubenschaft 24 in einen Pedikel einschraubbar, wobei ein Schraubendreher (nicht gezeichnet) in die als Innensechskant ausgebildeten Mittel 29 zur Aufnahme eines Schraubendrehers, welche endständig am Schraubenkopf 30 angeordnet sind, einführbar ist. Anstelle einer Ausführung als Innensechskant, können die Mittel 29 zur Aufnahme eines Schraubendreher beispielsweise auch als Innensechsrund, Torx oder Phillips ausgebildet sein.

Das Verbindungsteil 5 besteht aus einem zur Zentralachse 2 koaxialen Hohlkörper mit einem oberen Ende 6 und einem unteren Ende 7. Der Durchmesser des Hohlraumes 8 im Verbindungsteil 5 ist am unteren Ende 7 verengt, so dass ein Absatz 9 mit einer Auflagefläche 25 gebildet wird, worauf der Schraubenkopf 30 des Knochenfixationsmittels 1 auflegbar ist. Der Schraubenschaft 24 des Knochenfixationsmittels 1 ist durch den am unteren Ende 7 des Verbindungsteiles 5 verengten Hohlraum 8 durchführbar. Ferner wird das Verbindungsteil 5 senkrecht zur Zentralachse 2 von einem ersten Kanal 10 durchdrungen, wobei dieser erste Kanal 10 gegen das obere Ende 6 des Verbindungsteiles 5 offen ist. Die vom oberen Ende 6 des Verbindungsteiles 5 parallel zur Zentralachse 2 her gemessene Tiefe des Kanals 10 ist so ausgelegt, dass ein in den Kanal 10 eingelegter Längsträger 11 endständig auf dem Schraubenkopf 30 des Knochenfixationsmittels 1 auflegbar ist. Über das Verbindungsteil 5 geschoben ist die Verschlusskappe 12, wobei das vordere Ende 20 der Verschlusskappe 12 gegen das untere Ende 7 des Verbindungsteiles 5 gerichtet ist und das Verbindungsteil 5 parallel zur Zentralachse 2 im zweiten, in der Verschlusskappe 12 angebrachten Hohlraum 18 teilweise aufgenommen wird. Der die Verschlusskappe 12 senkrecht zur Zentralachse 2 durchdringende, zweite Kanal 17 gestattet die Aufnahme des im ersten Kanal 10 im Verbindungsteil 5 eingelegten Längsträgers 11 in der Verschlusskappe 12. Der zweite Kanal 17 ist am vorderen Ende 20 der Verschlusskappe 12 offen, so dass die Verschlusskappe 12 bei im Verbindungsteil 5 eingelegten Längsträger 11 parallel zur Zentralachse 2 über das Verbindungsteil 5 schiebbar ist.

Die Arretiermittel 21 sind parallel zur Zentralachse 2 betrachtet als peripher am Verbindungsteil 5 angebrachte Erhebungen 15 und dazu komplementäre, peripher im zweiten Hohlraum 18 der Verschlusskappe 12 angebrachte Vertiefungen 16 ausgebildet. Die Arretiermittel 21 sind in der gleichen Ausgestaltung auf zwei axialen Ebenen angeordnet, so dass verschiedene Rastenstellungen möglich sind. Vorzugsweise dient die erste Rastenstellung zur Reposition der zu fixierenden Knochen oder Knochenfragmente, während die zweite oder allfällige weitere Rastenstellungen zur Fixation der Vorrichtung dienen.

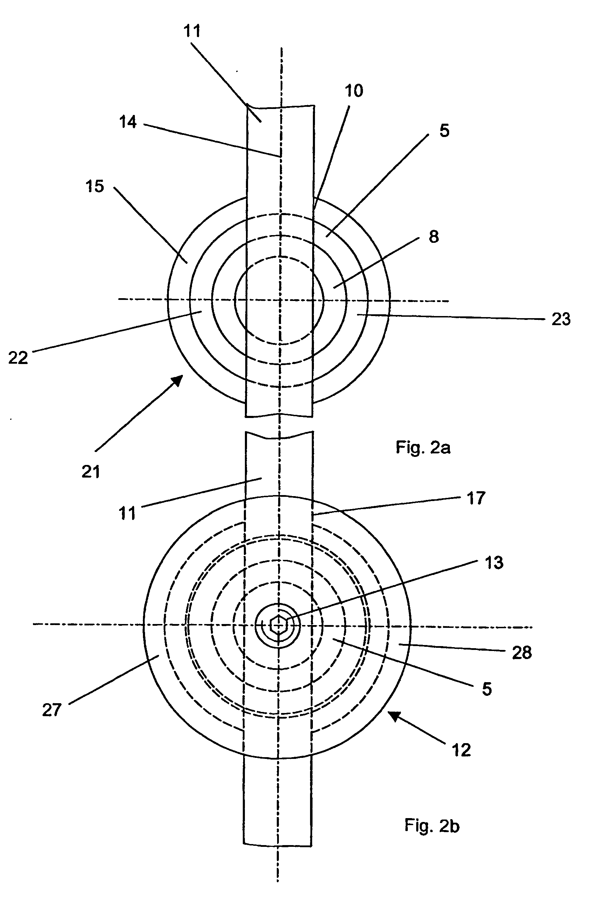

Die Erhebungen 15 und die Vertiefungen 16 weisen in einer zur Zentralachse 2 parallelen Querschnittsfläche ein sägezahnförmiges Profil auf, wobei die steilen Flanken der Erhebungen 15 gegen das untere Ende 7 des Verbindungsteiles 5 gerichtet sind und die steilen Flanken der Vertiefungen 16 gegen das hintere Ende 19 der Verschlusskappe 12 gerichtet sind. Wird die Verschlusskappe 12 parallel zur Zentralachse 2 über das Verbindungsteil 5 geschoben, federn die beiden durch den zweiten Kanal 17 gebildeten Segmente 27;28 (Fig. 2b) der Verschlusskappe 12 quer zur Zentralachse 2 aus, so dass die Verschlusskappe 12 über die Erhebungen 15 am Verbindungsteil 5 schiebbar sind bis die Vertiefungen 16 im zweiten Hohlraum 18 mit den Erhebungen 15 zur Deckung kommen und die beiden Segmente 27;28 (Fig. 2b) an der Verschlusskappe 12 gegen die Zentralachse 2 hin elastisch einschnappen.

Die Spannmittel 13 sind als Stiftschraube ausgeführt, welche in das Innengewinde in der zur Zentralachse 2 koaxialen Bohrung 31 am hinteren Ende 19 der Verschlusskappe 12 schraubbar ist.

Fig. 2 zeigt das Verbindungsteil 5 vom oberen Ende 6 her betrachtet. Der Hohlraum 8 und die Arretiermittel 21 sind zur Zentralachse 2 (Fig. 1) konzentrisch angeordnet, wobei die Arretiermittel 21 als Erhebungen 15 ausgebildet sind, welche parallel zur Zentralachse 2 betrachtet peripher am Verbindungsmittel 5 angebracht sind. Die ringförmige Seitenwand des Verbindungsteiles 5 sowie die Erhebungen 15 werden durch den Kanal 10 durchbrochen, wobei die Kanalachse 14 senkrecht zur Zentralachse 2 (Fig. 1) verläuft.

In den Kanal 10 eingelegt ist der Längsträger 11. Ferner werden durch den Kanal 10 am Verbindungsteil 5 auf einem Teil dessen Länge zwei Zungen 22;23 gebildet, wobei die Erhebungen 15 nur an diesen beiden Zungen 22;23 angebracht sind und nicht das gesamte Verbindungsteil 5 umschliessen.

Fig. 2b zeigt die auf das Verbindungsteil 5 montierte Verschlusskappe 12 mit dem Spannmittel 13. Durch den die Verschlusskappe 12 auf einem Teil ihrer Länge senkrecht zur Zentralachse 2 (Fig. 1) durchdringenden, zweiten Kanal 17 werden an der Verschlusskappe 12 zwei Segmente 27;28 gebildet, welche beim Überschieben der Verschlusskappe 12 über das Verbindungsteil 5 radial ausfedem, so dass die Verschlusskappe 12 über die Erhebungen 15 (Fig. 1) geschoben werden kann. So bald die Verschlusskappe 12 so weit über das Verbindungsteil 5 geschoben ist, dass die Vertiefungen 16 (Fig. 1) mit den Erhebungen 15 (Fig. 1) in Eingriff kommen können, federn die beiden Segmente 27;28 zurück, d.h. gegen die Zentralachse 2 (Fig. 1) hin.

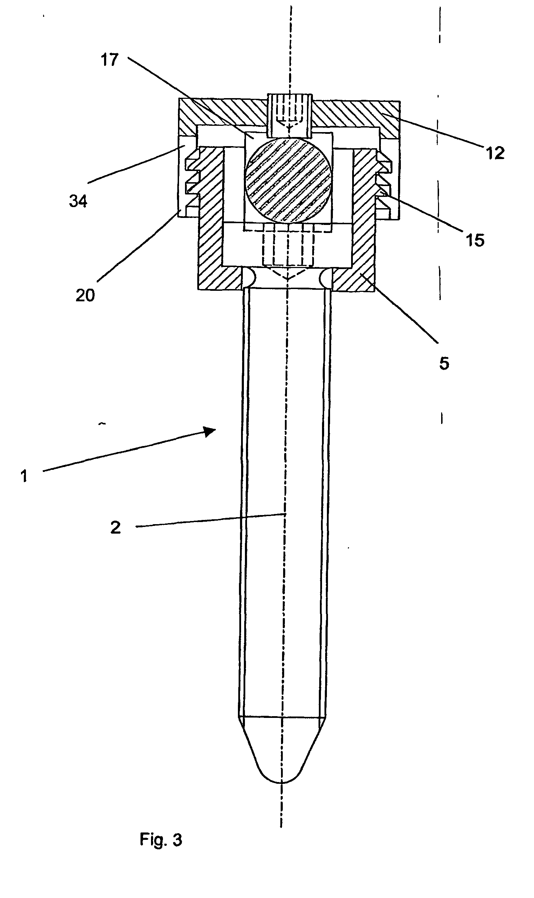

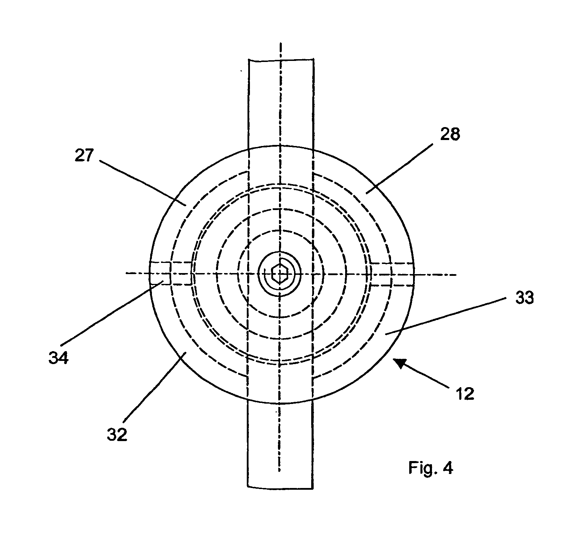

Die in den Fig. 3 und 4 dargestellte Ausführungsform der erfindungsgemässen Vorrichtung unterscheidet sich von der in den Fig. 1 und 2 dargestellten Ausführungsform nur darin, dass die Verschlusskappe 12 orthogonal zum zweiten Kanal 17 zwei Schlitze 34 aufweist, welche vom vorderen Ende 20 der Verschlusskappe 12 in deren Wand eingelassen sind. Anstelle der beiden Segmente 27;28 (Fig. 2b) weist die Verschlusskappe 12 in dieser Ausführungsform vier Segmente 27;28;32;33 auf. Dadurch ist erreichbar, dass die Elastizität der Verschlusskappe 12 erhöht wird und diese somit leichter über die Erhebungen 15 am Verbindungsteil 5 schiebbar ist.

Zur Implantation der Vorrichtung wird das vormontierte, das Knochenfixationsmittel 1 und das Verbindungsteil 5 umfassende Implantat mit einem in die Mittel 29 eingesetzten Schraubendreher aus dem Implantatbehälter entnommen, ohne dass ein weiteres Instrument für das Halten der Teile notwendig ist und ohne dass der Chirurg die Teile zusammenfügen muss. Anschliessend wird das vorläufig nur aus Knochenfixationsmittel 1 und Verbindungsteil 5 bestehende Implantat in den präparierten Pedikel eingedreht. Nach dem Einlegen des Längsträgers 11 in den im Verbindungsteil 5 angeordneten, ersten Kanal 10 wird mit demselben Schraubendreher die ebenfalls vormontierte, mit dem Spannmittel 13 versehene Verschlusskappe 12 aus dem lmplantatbehälter entnommen, wobei auch hier ein spezielles Halteinstrument entfällt. Mittels einer speziellen Klemme wird die Verschlusskappe 12 in der ersten Rastenstellung, d.h. wenn die erste Vertiefung 16 in der Verschlusskappe 12 über die erste Erhebung 15 am Verbindungsteil 5 geschoben wurde, verriegelt. Nach erfolgter Reposition wird die Verschlusskappe 12 in die zweite oder dritte Rastenstellung gebracht und verriegelt. Mit Hilfe des Schraubendrehers wird dann das Spannmittel 13 festgezogen und der im ersten Kanal 10 eingelegte Längsträger 11 im Verbindungsteil 5 blockiert. Beim Anziehen des Spannmittels 13 wird der Längsträger 11 auf den Schraubenkopf 30 gepresst, so dass dieser zwischen dem Absatz 9 und dem Längsträger 11 eingespannt wird und das Knochenfixationsmittel 1 zusammen mit dem Längsträger 11 im Verbindungsteil 5 blockiert wird.

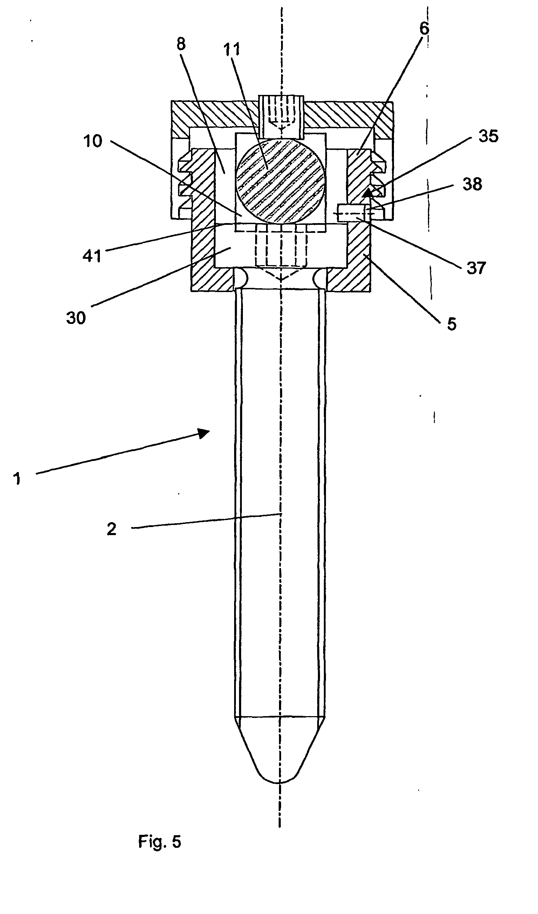

In Fig. 5 ist eine Ausführungsform der erfindungsgemässen Vorrichtung dargestellt, welche sich von den oben beschriebenen Ausführungsformen nur darin unterscheidet, dass der Schraubenkopf 30 des Knochenfixationsmittels 1 durch Sicherungsmittel 35 gegen ein Ausfahren aus dem Hohlraum 8 gesichert ist. Die Sicherungsmittel 35 umfassen einen Stift 37, welcher in einer quer zur Zentralachse 2 verlaufenden Bohrung 38 beispielsweise eingepresst ist und in den Hohlraum 8 hineinragt. Axial ist die Bohrung 38 zwischen dem hinteren Ende 41 des Schraubenkopfes 30 und dem oberen Ende 6 des Verbindungsteiles 5 angeordnet. Anstelle einer Bohrung 38 können auch mehrere auf dem Umfang verteilte Bohrungen 38 und mehrere Stifte 37 als Sicherungsmittel 35 vorgesehen werden.

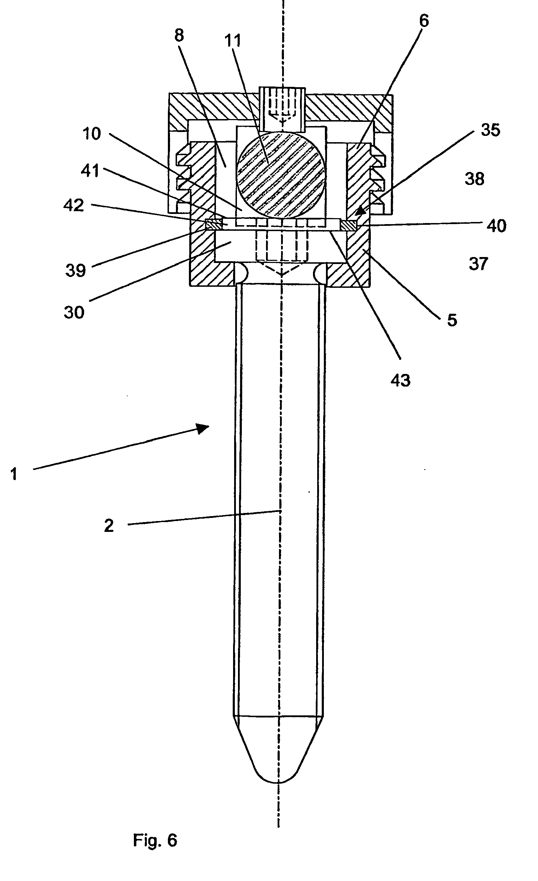

Fig. 6 zeigt eine Ausführungsform der erfindungsgemässen Vorrichtung, worin die Sicherungsmittel 35 durch einen Sprengring 39 anstatt durch einen oder mehrere Stifte 37 ausgeführt werden. Der Sprengring 39 wird in einer Nute 40, welche beim hinteren Ende 41 des Schraubenkopf 30 angeordnet ist, aufgenommen und ragt in den Hohlraum 8 hinein, so dass dort der Hohlraum 8 gegen sein oberes Ende 6 verengt wird. Die Nute 40 ist axial so angeordnet, dass der darin eingefügte Sprengring 39 das hintere Ende 41 des Schraubenkopfes 30 gegen das obere Ende 6 des Verbindungsteiles 5 nicht überragt, so dass der Längsträger 11 nicht auf dem Sprengring 39 aufliegt. Der Schraubenkopf 30 ist dazu beispielsweise bei seinem hinteren Ende 41 abgesetzt ausgestaltet und weist bei seinem hinteren Ende 41 ein axiales Segment 42 auf, welches einen kleineren Durchmesser aufweist als der Schraubenkopf 30. Die durch dieses im Durchmesser verringerte Segment 42 gebildete Schulter 43 ist an den als Sprengring 39 ausgebildeten Sicherungsmitteln 35 zur Anlage bringbar, so dass der Schraubenkopf 30 durch den Sprengring 39 im Hohlraum 8 gegen axiale Verschiebungen gegen das obere Ende 6 des Verbindungsteiles 5 arretiert ist. Das Segment 42 des Schraubenkopfes 30 geht durch ringförmige Öffnung des Sprengringes 39 hindurch und ragt endständig über den Sprengring 39 hinaus, so dass der Längsträger 11 am hinteren Ende 41 des Schraubenkopfes 30 aufliegen kann.

## Patentansprüche

1. Vorrichtung zur Verbindung eines Längsträgers (11) mit einem Knochenfixationsmittel (1), insbesondere einer Pedikelschraube umfassend
A) ein koaxial zur Zentralachse (2) angeordnetes Verbindungsteil (5), mit einem oberen Ende (6), einem unteren Ende (7), einem zur Zentralachse (2) koaxialen, das Verbindungsteil (5) vom oberen Ende (6) bis zum unteren Ende (7) durchdringenden Hohlraum (8), welcher mittels mindestens eines Absatzes (9) gegen das untere Ende (7) verjüngt ausgebildet ist, und einem das Verbindungsteil (5) quer zur Längsachse (2) durchdringenden Kanal (10) zur Aufnahme eines Längsträgers (11);
B) eine Verschlusskappe (12), welche ein vorderes Ende (20), ein hinteres Ende (19), einen zweiten, am vorderen Ende (20) offenen Hohlraum (18) zur Aufnahme des Verbindungsteiles (5) und einen zweiten, quer zur Zentralachse (2) gegen das vordere Ende (20) der Verschlusskappe (12) offenen Kanal (17) umfasst; und
C) Spannmittel (13), welche am hinteren Ende (19) der Verschlusskappe (12) mit dieser verbindbar sind und mittels welcher ein im Kanal (10) eingelegter Längsträger (11) im Verbindungsteil (5) fixierbar ist, wobei
D) aussen am Verbindungsteil (5) und im zweiten Hohlraum (18) in der Verschlusskappe (12) zueinander komplementäre, einschnappbare Arretiermittel (21) angebracht sind, welche zur Befestigung der Verschlusskappe (12) am Verbindungsteil (5) dienen,
**dadurch gekennzeichnet, dass**
E) die Vorrichtung Sicherungsmittel (35) umfasst, wodurch der Schraubenkopf (30) eines Knochenfixationsmittels (1) gegen ein Ausfahren aus dem Hohlraum (8) gesichert wird.

2. Vorrichtung nach Anspruch 1, **dadurch gekennzeichnet, dass** die Arretiermittel (21) in einer zur Zentralachse (2) orthogonalen Querschnittsfläche auf der Peripherie des Verbindungsteiles (5) und auf der Peripherie des zweiten Hohlraumes (18) in der Verschlusskappe (12) angeordnet sind.

3. Vorrichtung nach Anspruch 2, **dadurch gekennzeichnet, dass** die Arretiermittel (21) Erhebungen (15) aussen am Verbindungsteil (5) und komplementäre Vertiefungen (16) im zweiten Hohlraum (18) in der Verschlusskappe (12) umfassen.

4. Vorrichtung nach einem der Ansprüche 1 bis 3, **dadurch gekennzeichnet, dass** der Absatz (9) eine ebene, zur Zentralachse (2) konzentrische kreisringförmige Auflagefläche (25) umfasst.

5. Vorrichtung nach einem der Ansprüche 1 bis 4, **dadurch gekennzeichnet, dass** die Verschlusskappe (12) zwei zum zweiten Kanal (17) orthogonal angeordnete Schlitze (34) umfasst, welche vom vorderen Ende (20) her in die Wand der Verschlusskappe (12) eindringen.

6. Vorrichtung nach einem der Ansprüche 1 bis 5, **dadurch gekennzeichnet, dass** sie ein Knochenfixationsmittel (1) mit einer Zentralachse (2), einem vorderen Segment (3) und einem axial angrenzenden hinteren Segment (4) umfasst, wobei das hintere Segment (4) zylindrisch oder prismatisch ausgebildet ist und das vordere Segment (3) zur Fixation an einem Knochen dient, und dass das hintere Segment (4) des Knochenfixationsmittels (4) durch den Absatz (9) axial gegen das untere Ende (7) des Verbindungsteils (5) arretierbar ist während das vordere Segment (3) des Knochenfixationsmittels (1) am unteren Ende (7) des Verbindungsteils (5) axial hervorsteht.

7. Vorrichtung nach Anspruch 6, **dadurch gekennzeichnet, dass** das Knochenfixationsmittel (1) eine Pedikelschraube mit einem ein Aussengewinde (26) aufweisenden Schraubenschaft (24) und einem endständigen Schraubenkopf (30) ist.

## Revendications

1. Dispositif permettant d'assembler un support longitudinal (11) à un moyen de fixation d'ostéosynthèse (1), notamment à une vis pédiculaire, ledit dispositif comprenant :
A) un élément d'assemblage (5) disposé de manière coaxiale à l'axe central (2) et comportant une extrémité supérieure (6), une extrémité inférieure (7), un espace vide (8) disposé coaxialement à l'axe central (2) et traversant l'élément d'assemblage (5) depuis son extrémité supérieure (6) jusqu'à son extrémité inférieure (7), lequel se rétrécit vers l'extrémité inférieure (7) au moyen d'au moins un épaulement (9), un canal (10) traversant l'élément d'assemblage (5) transversalement à l'axe central (2) et permettant de recevoir un support longitudinal (11);
B) un chapeau-obturateur (12) qui comprend une extrémité avant (20), une extrémité arrière (19), un deuxième espace vide (18) ouvert à l'extrémité avant (20) et permettant de recevoir l'élément d'assemblage (5) ainsi qu'un deuxième canal (17) ouvert, transversalement à l'axe central (2), vers l'extrémité avant (20) du chapeau-obturateur (12); et
C) des moyens de serrage (13) pouvant être reliés au chapeau-obturateur (12) à l'extrémité arrière (19) de celui-ci et permettant de fixer dans l'élément d'assemblage (5) un support longitudinal (11) inséré dans le canal (10),
D) des moyens d'arrêt (21) complémentaires et pouvant s'enclencher les uns dans les autres étant formés respectivement à l'extérieur de l'élément d'assemblage (5) et dans le deuxième espace vide (18) ménagé dans le chapeau-obturateur (12), lesquels servent à fixer le chapeau-obturateur (12) à l'élément d'assemblage (5),
**caractérisé en ce que**
E) le dispositif comprend des moyens de retenue (35) empêchant la tête de vis (30) d'un moyen de fixation d'ostéosynthèse (1) de sortir de l'espace vide (8).

2. Dispositif selon la revendication 1, **caractérisé en ce que** les moyens d'arrêt (21) sont disposés, dans une surface de coupe transversale s'étendant orthogonalement à l'axe central (2), sur la périphérie de l'élément d'assemblage (5) et sur la périphérie du deuxième espace vide (18) ménagé dans le chapeau-obturateur (12).

3. Dispositif selon la revendication 2, **caractérisé en ce que** les moyens d'arrêt (21) comprennent des saillies (15) formées extérieurement sur l'élément d'assemblage (5) et des creux (16) complémentaires formés dans le deuxième espace vide (18) ménagé dans le chapeau-obturateur (12).

4. Dispositif selon l'une des revendications 1 à 3, **caractérisé en ce que** l'épaulement (9) comprend une surface d'appui plane (25) en forme d'anneau de cercle, concentrique à l'axe central (2).

5. Dispositif selon l'une des revendications 1 à 4, **caractérisé en ce que** le chapeau-obturateur (12) comprend deux fentes (34) disposées orthogonalement au deuxième canal (17), lesquelles pénètrent dans la paroi du chapeau-obturateur (12) à partir de l'extrémité avant (20).

6. Dispositif selon l'une des revendications 1 à 5, **caractérisé en ce qu'**il comprend un moyen de fixation d'ostéosynthèse (1) présentant un axe central (2), un segment avant (3) et un segment arrière (4) contigu de manière axiale, le segment arrière (4) étant de forme cylindrique ou prismatique et le segment avant (3) servant à la fixation sur un os, et **en ce que** le segment arrière (4) du moyen de fixation d'ostéosynthèse (1) pouvant être arrêté axialement par l'épaulement (9) contre l'extrémité inférieure (7) de l'élément d'assemblage (5) alors que le segment avant (3) du moyen de fixation d'ostéosynthèse (1) dépasse axialement de l'extrémité inférieure (7) de l'élément d'assemblage (5).

7. Dispositif selon la revendication 6, **caractérisé en ce que** le moyen de fixation d'ostéosynthèse (1) est une vis pédiculaire constituée par un corps de vis (24) présentant un filetage extérieur (26) et une tête de vis (30) disposée à l'extrémité de celui-ci.

## Claims

1. Device for connecting a longitudinal carrier (11) to a bone fixation means (1), particularly a pedicle screw comprising
A) a connection element (5) arranged coaxial to the central axis (2), with an upper end (6), a lower end (7), a cavity (8) coaxial to the central axis (2) passing through the connection element (5) from the upper end (6) to the lower end (7), which is designed tapering towards the lower end (7) by means of at least one shoulder (9), and a channel (10) passing through the connection element (5) transversely to the longitudinal axis (2) for receiving a longitudinal carrier (11);
B) a sealing cap (12), comprising a front end (20), a rear end (19), a second cavity (18) open at the front end (20) for receiving the connection element (5) and a second channel (17) transversely to the central axis (2) and open towards the front end (20) of the sealing cap (12); and
C) tensioning means (13), which are connectable to the sealing cap (12) at its rear end (19) and by means of which a longitudinal carrier (11) inserted in the channel (10) can be fixed in the connection element (5), whereby
D) externally on the connection element (5) and in the second cavity (18) in the sealing cap (12) latch-in arresting means (21) are arranged complementary to each other, which serve for securing the sealing cap (12) to the connection element (5),
**characterised in that**
E) the device comprises securing means (35) wherewith the screw head (30) of a bone fixation means (1) is secured from driving out of the cavity 8.

2. Device according to Claim 1, **characterised in that** the arresting means (21) are arranged in a cross-section surface orthogonal to the central axis (2) on the periphery of the connection element (5) and on the periphery of the second cavity (18) in the sealing cap (12).

3. Device according to Claim 2, **characterised in that** the arresting means (21) comprise bulges (15) externally on the connection element (5) and complementary depressions (16) in the second cavity (18) in the sealing cap (12).

4. Device according to one of the claims 1 to 3, **characterised in that** the shoulder (9) comprises a level bearing surface (25) of circular-ring shape concentric to the central axis (2).

5. Device according to one of the claims 1 to 4, **characterised in that** the sealing cap (12) comprises two slots (34) arranged orthogonal to the second channel (17), which penetrate the wall of the sealing cap (12) from the direction of the front end (20).

6. Device according to one of the claims 1 to 5, **characterised in that** it comprises a bone fixation means (1) with a central axis (2), a front segment (3) and an axially adjoining rear segment (4), wherein the rear segment (4) has a cylindrical or prismatic form and the front segment (3) is used for fixation to a bone, and that the rear segment (4) of the bone fixation means (4) is arrested by the shoulder (9) axial towards the lower end (7) of the connection element (5) whereas the front segment (3) of the bone fixation means (1) protrudes axially at the lower end (7) of the connection element (5).

7. Device according to Claim 6, **characterised in that** the bone fixation means (1) is a pedicle screw with a screw shaft (24) having an external thread (26) and a screw head (30) at the end position.
